(19) Europäisches Patentamt
European Patent Office
Office européen des brevets

(11) **EP 2 694 267 B2**

(12) **NEW EUROPEAN PATENT SPECIFICATION**
After opposition procedure

(45) Date of publication and mention of the opposition decision:
**11.03.2020   Bulletin 2020/11**

(45) Mention of the grant of the patent:
**15.03.2017   Bulletin 2017/11**

(21) Application number: **12717517.2**

(22) Date of filing: **05.04.2012**

(51) Int Cl.:
*A61K 8/34* (2006.01)   *A61Q 5/02* (2006.01)
*A61K 8/73* (2006.01)   *A61K 8/81* (2006.01)
*A61Q 19/10* (2006.01)   *A61K 8/02* (2006.01)
*A61K 8/04* (2006.01)   *B29B 7/46* (2006.01)
*B29C 41/28* (2006.01)   *B29C 47/76* (2006.01)
*B29C 47/88* (2006.01)   *B29C 67/20* (2006.01)
*B29C 47/92* (2006.01)   *B29K 105/04* (2006.01)
*B29C 47/00* (2006.01)   *B29C 47/78* (2006.01)
*C08J 9/30* (2006.01)

(86) International application number:
**PCT/US2012/032253**

(87) International publication number:
**WO 2012/138820 (11.10.2012 Gazette 2012/41)**

(54) **CONTINUOUS PROCESS OF MAKING AN ARTICLE OF DISSOLUTION UPON USE TO DELIVER SURFACTANTS**

KONTINUIERLICHES VERFAHREN ZUR HERSTELLUNG EINES SICH BEI VERWENDUNG AUFLÖSENDEN ARTIKELS ZUR FREISETZUNG VON TENSIDEN

PROCÉDÉ EN CONTINU PERMETTANT DE FABRIQUER UN ARTICLE DESTINÉ À SE DISSOUDRE EN COURS D'UTILISATION POUR LIBÉRER DES TENSIOACTIFS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:   **07.04.2011   US 201161472941 P**

(43) Date of publication of application:
**12.02.2014   Bulletin 2014/07**

(73) Proprietor: **The Procter & Gamble Company**
**Cincinnati, OH 45202 (US)**

(72) Inventors:
• **GLENN, Robert, Wayne, Jr.**
  **Liberty Township, OH 45044 (US)**
• **GRANBERG, Eric, Paul**
  **Cincinnati, OH 45240 (US)**
• **THOMPSON, Todd, Ryan**
  **Cincinnati, Ohio 45202 (US)**
• **QUAN, Ke-ming**
  **West Chester, OH 45069 (US)**
• **HECHT, John, Philip**
  **Cincinnati, Ohio 45202 (US)**
• **MCCARTY, Jason, Donald**
  **Wilmington, OH 45177 (US)**
• **NUNES, Raul, Victorino**
  **Loveland, OH 45140 (US)**
• **PINYAYEV, Aleksey, Mikhailovich**
  **West Chester, OH 45069 (US)**

(74) Representative: **P&G Patent Germany**
**Procter & Gamble Service GmbH**
**Sulzbacher Straße 40**
**65824 Schwalbach am Taunus (DE)**

(56) References cited:
WO-A2-2010/077627   DE-A1- 2 925 915
US-A- 2 687 877   US-A- 5 976 454
US-A1- 2002 091 169   US-A1- 2010 298 188
US-A1- 2011 027 328

• Datenblatt "AeroDry Impinge ment Ovens, Roasters and Dryers".

Remarks:
The file contains technical information submitted after the application was filed and not included in this specification

**Description**

**FIELD OF THE INVENTION**

The present invention relates to a continuous process for making a flexible porous dissolvable solid structure article as a personal care product.

**BACKGROUND OF THE INVENTION**

Dissolvable porous solid personal care products have been disclosed e.g. in US2011/0027328, comprising a water-soluble polymeric structurant and a surfactant or other ingredient. However, existing processes for making these dissolvable porous solid structures have less optimal cost, rate of manufacture, and product variability parameters.
A need still exists for a process that results in a desired flexible, dissolvable porous solid structure which can be manufactured within the desired cost and rate parameters. Furthermore, a need exists for a process that results in a dissolvable porous solid structure with a faster drying time, and uniform consistency in the open celled foam of the dissolvable porous solid structure.

**SUMMARY OF THE INVENTION**

The present invention relates to a continuous process as defined in the claims.

**BRIEF DESCRIPTION OF THE DRAWINGS**

Fig. 1 is an exemplary embodiment of the equipment used for practicing a continuous process for generating an Article.
Fig. 2 is a cross sectional view of five stacked Articles made by a continuous process.
Fig. 3 is a cross sectional view of a product made by a batch process.
Fig. 4 is a cross sectional view of a product made by a batch process.

**DETAILED DESCRIPTION OF THE INVENTION**

In all embodiments of the present invention, all percentages are by weight of the total composition, unless specifically stated otherwise. All ratios are weight ratios, unless specifically stated otherwise. Unless otherwise indicated, all measurements are understood to be made at 25°C and at ambient conditions, where "ambient conditions" means conditions under about one atmosphere of pressure and at about 50% relative humidity. All such weights as they pertain to listed ingredients are based on the active level and do not include carriers or by-products that may be included in commercially available materials, unless otherwise specified.

**Definitions**

The flexible porous dissolvable solid structure article may be referred to herein as "the Article" or "the Dissolvable Article". All references are intended to mean the flexible dissolvable porous solid structure article.
As used herein, "flexible" means that the porous dissolvable solid structure article meets the distance to maximum force values discussed herein.
As used herein, "dissolvable" means that the flexible porous dissolvable solid structure article meets the hand dissolution values discussed herein. The Article has a hand dissolution value of from about 1 to about 30 strokes, in one embodiment from about 2 to about 25 strokes, in another embodiment from about 3 to about 20 strokes, and in still another embodiment from about 4 to about 15 strokes as measured by the Hand Dissolution Method.
As used herein "open celled foam" means a solid, interconnected, polymer-containing matrix that defines a network of spaces or cells that contain a gas, typically a gas such as air, without collapse of the foam structure during the drying process, thereby maintaining the physical strength and cohesiveness of the solid. The interconnectivity of the structure may be described by a Star Volume, a Structure Model Index (SMI) and a Percent Open Cell Content.
As used herein, the articles including "a" and "an" when used in a claim, are understood to mean one or more of what is claimed or described.
As used herein, the terms "include," "includes," and "including," are meant to be non-limiting.
The test methods disclosed in the Test Methods Section of the present application should be used to determine the respective values of the parameters of Applicants' inventions.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

It has been unexpectedly found that an Article produced according to the continuous process disclosed herein results in more uniform and consistent structures in the open celled foam of the Article. Conventional batch processing techniques can result in Articles comprising distinct regions: an upper region that is closest to the target density, a middle region with a significantly lower density and larger pores, and a bottom region with a higher density and thicker cell walls. This higher density bottom region may negatively impact the flow of water through the Article and may result in slower dissolution. In addition, the higher density bottom region may be the rate limiting step for drying the Article.

In contrast, the open-celled porous structures produced by the continuous process herein have improved uniformity and consistency in the regions of the Article. Due to the uniformity of bubble sizes in the open celled foam, regions are produced with a uniform density. This allows for faster manufacture of the Article, faster drying of the Article, and faster dissolution in use.

## Method of Manufacture

The Article can be prepared by the continuous process as defined in the claims.

### A. Preparation of Pre-Mixture

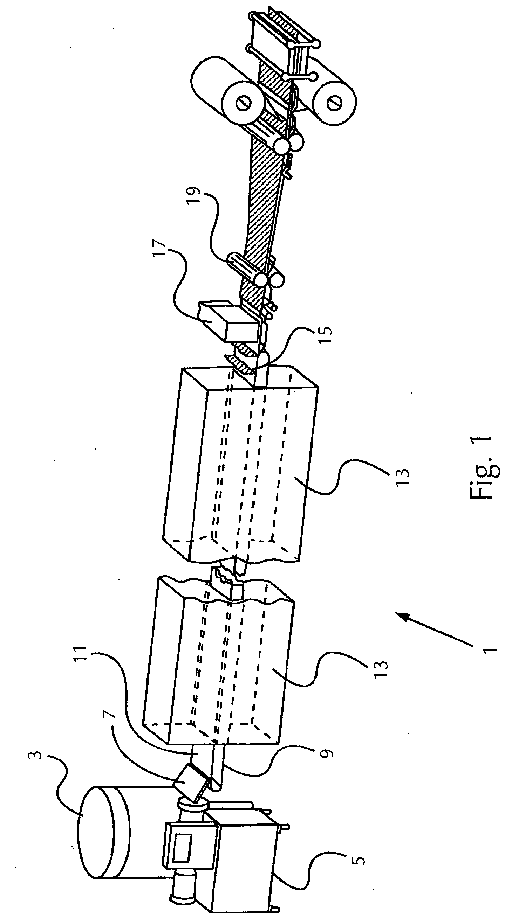

Fig. 1 depicts an exemplary embodiment of the equipment useful for practicing a continuous process for generating an Article. As shown in Fig. 1, in the continuous manufacturing process for an Article 1 the solids of interest are mixed in a premix tank 3. The pre-mixture is generally prepared by mixing the solids of interest, including surfactant(s), dissolved water soluble polymer, optional plasticizer and other optional ingredients. The solids of interest are present in the pre-mixture at a level by weight of from about 1% to about 75% surfactant, from about 0.1% to about 25% water soluble polymer, and from about 0.1% to about 25% plasticizer.

In one embodiment, the pre-mixture can be formed using a mechanical mixer. Mechanical mixers useful herein, include, but aren't limited to pitched blade turbines or MAXBLEND mixer (Sumitomo Heavy Industries).

For addition of the ingredients in the pre-mixture, it can be envisioned that the polymer is ultimately dissolved in the presence of water, the surfactant(s), optional actives, optional plasticizer, and any other optional ingredients including step-wise processing via pre-mix portions of any combination of ingredients.

The pre-mixtures of the present invention comprise: from about 15% to about 55% solids, in one embodiment from about 30% to about 55% solids, in one embodiment from about 32% to about 55% solids, in one embodiment from about 34% to about 50%, and in another embodiment from about 36% to about 45% solids, by weight of the pre-mixture before drying. The percent solids content is the summation of the weight percentages by weight of the total processing mixture of all of the solid, semi-solid and liquid components excluding water and any obviously volatile materials such as low boiling alcohols.

In one embodiment, the viscosity of the pre-mixture is determined when the pre-mixture is heated to a temperature in the range of from about 60°C to about 99°C. The viscosity is measured at at a shear rate of 1 sec$^{-1}$ and 70°C. In another embodiment, the viscosity of the pre-mixture is measured at ambient temperatures (25 °C).

When the pre-mixture is heated to a temperature in the range of between 60°C and 99°C, the pre-mixtures of the present invention have a viscosity of from about 1000 cps to about 20,000 cps. The pre-mixture viscosity values are measured using a Brookfield RVDV-1 Prime Viscometer with CPE-41 cone and a shear rate of 1.0 reciprocal seconds for a period of 300 seconds.

### B. Optional Continued Heating of Pre-Mixture

Optionally, the pre-mixture is pre-heated immediately prior to the aeration process at above ambient temperature but below any temperatures that would cause degradation of the component. The pre-mixture is kept at 60°C to 90°C. In one embodiment, when the viscosity at ambient temperature of the pre-mix is from about 1000 cps to about 20,000 cps, the optional continuous heating is utilized before the aeration step. In an additional embodiment, additional heat is applied during the aeration process to try and maintain an elevated temperature during the aeration. This can be accomplished via conductive heating from one or more surfaces, injection of steam or other processing means.

It is believed that the act of pre-heating the pre-mixture before the aeration step may provide a means for lowering the viscosity of pre-mixtures comprising higher percent solids content for improved introduction of bubbles into the mixture and formation of the desired Article. Achieving higher percent solids content is desirable so as to reduce the energy requirements for drying. The increase of percent solids, and therefore conversely the decrease in water level content, and increase in viscosity is believed to affect the bubble drainage from the pre-mixture during the drying step. The drainage and evaporation of water from the pre-mixture during drying is believed to assist the formation of the open celled structure of the Article.

Pre-heating of the pre-mixture also allows for the manufacture of a fast dissolving Article even when using a more viscous processing mixture. Without pre-heating, these viscous processing mixtures with higher percent solid levels normally produce Articles that are slow dissolving and that have predominately closed celled foams. However, the increased temperature during pre-heating causes bubble drainage from the thin film bubbles facing outwards into the plateau borders of the open celled foam. This drainage generates openings between the bubbles which become the open cells of the Article. The demonstrated ability to achieve such inter-connected open-celled foams of the Articles of the present invention is surprising.

In addition, a more viscous processing mixture results in Articles with low percent (%) shrinkage after the drying process while still maintaining fast dissolution rates. This is due to the fact that during the drying process, pre-mixtures with higher viscosities are able to mitigate the drainage and bubble rupture/collapse/coalescence that give rise to the shrinkage.

## C. Aeration of Pre-Mixture

The aeration of the pre-mixture is accomplished by introducing a gas into the pre-mixture in one embodiment by mechanical mixing energy but also may be achieved via chemical means to form an aerated mixture. As shown in Fig. 1, the aeration of the pre-mixture is achieved by an aeration unit 5. The aeration is accomplished by using a roter stater mixer.

The unique design of the high shear rotor/stator mixing head may lead to uniform bubble sizes in the layers of the open celled foam.

Bubble size of the wet aerated pre-mixture assists in achieving uniform layers in the open celled foam. In one embodiment, the bubble size of the wet aerated pre-mixture is 20 microns to 80 microns.

The uniformity of the bubble sizes causes the Article to have consistent densities in the layers of the Article. The wet aerated pre-mixture has a density from 0.15 to 0.65 g/mol.

## D. Forming the Aerated Wet Pre-Mixture

As seen in Fig. 1, the forming of the aerated wet pre-mixture is accomplished by extruding the aerated mixture through an extrusion nozzle 7 onto a continuous belt 9 or screen comprising any non-interacting or non-stick material such as solid metallic materials, flexible plastic materials including infrared transparent materials, and combinations thereof. Nonlimiting examples of solid metallic materials include stainless steel. Nonlimiting examples of flexible plastic materials including but are not limited to materials such as HDPE, polycarbonate, NEOPRENE®, rubber, LDPE, and fiberglass. Nonlimiting examples of infrared transparent materials include but are not limited to TEFLON®.

After extrusion, the aerated wet pre-mixture forms one or more sheets. In one embodiment, one sheet 11 forms the Article having a thickness of from about 5mm to about 10mm. In another embodiment, the Article has a thickness of about 6.5mm. In another embodiment, two or more sheets 11 are combined to form an Article having a final thickness from about 5mm to about 10mm. The extrusion of thinner sheets that are then combined to form an Article allows for a faster drying time for the individual sheets. The sheets can be combined by any means known in the art, examples of which include but are not limited to, chemical means, mechanical means, and combinations thereof. The combination of the sheets allows for two or more sheets to be stacked on top of one another.

The wet density range of the aerated pre-mixture ranges from about 0.15 g/cm$^3$ to about 0.65 g/cm$^3$, from about 0.20 g/cm$^3$ to about 0.45 g/cm$^3$, from about 0.25 g/cm$^3$ to about 0.40 g/cm$^3$, and from about 0.30 g/cm$^3$ to about 0.35 g/cm$^3$.

The sheets have an average pore size of from about 150 microns to about 450 microns, in one embodiment from about 175 microns to about 350 microns, and in another embodiment from about 200 microns to about 300 microns. The sheets also have an average cell wall thickness of from about 15 microns to about 55 microns, in one embodiment from about 20 microns to about 45 microns, and in another embodiment from about 25 microns to about 35 microns. The sheets of the Article also have an average top surface bubble size of from about 20 microns to about 250 microns, in another embodiment from about 40 microns to about 220 microns, in another embodiment from about 60 microns to about 190 microns, and in another embodiment from about 100 microns to about 160 microns.

### E. Drying the Formed Aerated Wet Pre-Mixture

Various processes for drying aerated wet pre-mixtures, and exemplary methods and materials are set forth in U.S. Patent Application No. 12/633,228.

The drying environment 13 is an impingement oven, such that the drying environment is between 100°C and 150°C.

As seen in Fig. 1, the drying environment is such that the formed Article 15 has a moisture content of from about 5% to about 10% and a dry density of from about 0.10 g/cm$^3$ to about 0.40 g/cm$^3$.

The drying environment is heated to a temperature between 100°C and 150°C. In one embodiment, the drying temperature is between 105°C and 145°C. In another embodiment, the drying temperature is between 110°C and 140°C. In a further embodiment, the drying temperature is between 115°C and 135°C.

After drying, one sheet forms the Article having a thickness of from about 5mm to about 10mm. In another embodiment, the Article has a thickness of about 6.5mm. In another embodiment, two or more sheets are combined to form an Article having a final thickness from about 5mm to about 10mm. The extrusion of thinner sheets that are then combined to form an Article allows for a faster drying time for the individual sheets.

In one embodiment, the drying time for full thickness of the Article (10mm) is from about 3 minutes to about 90 minutes, in another embodiment from about 5 minutes to about 60 minutes, in another embodiment from about 7 minutes to about 45 minutes. The drying step results in the Article.

The resulting Article (dried) may comprise a dry density of from about 0.10 g/cm$^3$ to about 0.40 g/cm$^3$, in one embodiment from about 0.11 g/cm$^3$ to about 0.30 g/cm$^3$, in another embodiment from about 0.12 g/cm$^3$ to about 0.25 g/cm$^3$, and in another embodiment from about 0.13 g/cm$^3$ to about 0.20 g/cm$^3$.

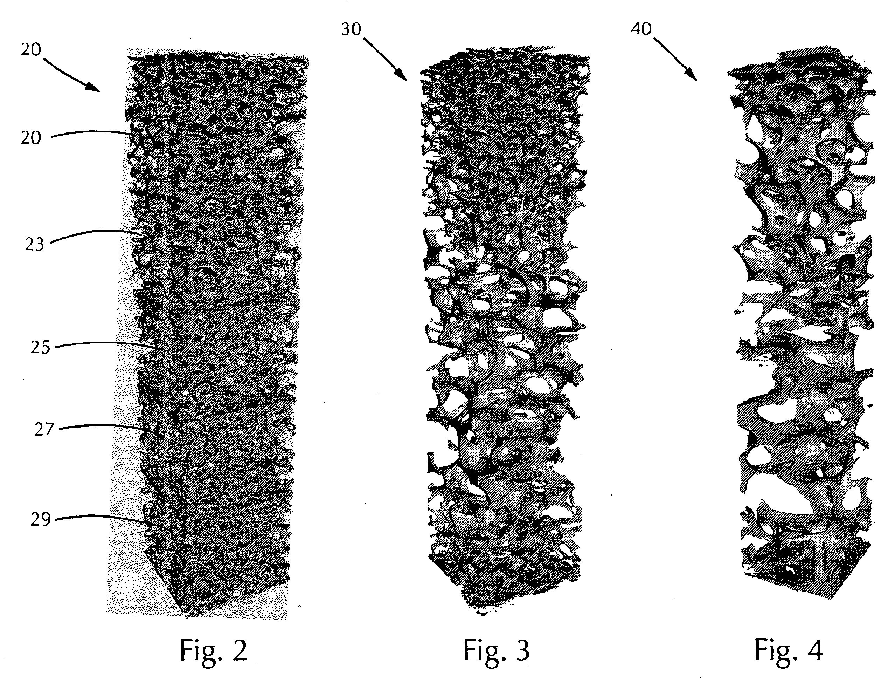

The resulting Article also has an open celled foam with a percent open cells of from about 80% to about 100%. It has been unexpectedly found that the Article produced by the continuous process has uniformity in the upper, middle, and lower regions of the open celled foam. In one embodiment, the Articles are stacked in layers. Fig. 2 is five stacked Articles with Article 20 comprising five layers 21, 25, 27, and 29, each with uniformity in the regions of open celled foam. In contrast, Fig. 3 shows the product of a batch process 30 with an Oakes mixer that produces non-uniform regions in the open celled foam of the product. Fig. 4 shows the product of a batch process 40 with a Kitchen Aid that produces non-uniform regions in the open celled foam of the product.

### F. Further Optional Steps

Further optional steps not recited above may be added at any point during or after the recited process. Optional ingredients may be imparted during any of the above described four processing steps or even after the drying process. As seen in Fig. 1, further optional steps may include further finishing steps 17, such as the addition of heat sensitive materials including but not limited to perfumes and enzymes; cutting of the Article into a smaller size by a cutting knife 19; puncturing or slitting the Article, further manipulation of the Article such as forming a three-dimensional shape, printing, texturizing, mixing the Article into another composition, laminating the Article with another material, packaging the Article and other process steps.

Additional steps that can be used in the present process include cutting the resulting Article into smaller sizes, puncturing the Article with needles or slitting the Article. The size of the Article will depend upon the desired dosage amount of actives, or in this case surfactant. The frequency of perforation or slitting is confined to maintain the structural integrity of the Article such that it can still be handled.

The Article may be further manipulated into a shape or form other than a flat plane or sheet. Other three-dimensional shapes may include spherical bead or ball, flowers, flower petals, berry shapes and various known pasta shapes. As such the process may further include a step whereby the Article is manipulated into a three-dimensional shape.

The Article may undergo different manipulation such as being printed upon or texturized by dimpled, waffled or otherwise topographically patterned surfaces including letters, logos or figures. The printing or texturizing of the Article can also be the result of creping processes, imprinted coatings, embossing patterns, laminating to other layers having raised portions, or the result of the physical form of the dissolvable porous solid substrate itself. The printing step may include printing by spraying, knife, rod, kiss, slot, painting, printing such as flexographic (flexo) printing and combinations thereof. Therefore, the present process can further include the step of printing or texturing the Article.

The Article may be utilized by being mixed with other compositions or products. The mixing should not detract from the dissolution properties herein described. Therefore the present process may further comprise the step of mixing the Article with another composition, mixing the Article with another product.

The Article may be packaged for consumption individually or in a plurality of Articles. The Article may be included in a kit wherein various types of products are supplied, including Articles with different compositions, Article(s) with other products making up a regime of series of products for a desired benefit, or Article(s) with other products unrelated such as a toiletry travel kit for travel on airplanes.

Suitable packaging material may be selected such that the Article is protected from inadvertent exposure to liquids. The packaging material may be air and/or vapor permeable, dependent upon the environment in which the Article is to be sold.

The process may further include a step of packaging the Article individually for sale as a product. The process may further include a step of packaging a plurality of Article for sale as a product. The process may further include a step of including a packaged Article in a kit for sale as a product. The packaging step is undertaken after the formation of the Article, in one embodiment after the Article is cut into a suitable size. The Article may be packaged on the same line as the production of Article or the Article may be collected, shipped or stored, and then packaged at a later time.

## Composition of Article

## A. Surfactants

The Article comprises one or more surfactants suitable for application to the hair or skin. Surfactants suitable for use in the Article include anionic surfactants, nonionic surfactants, cationic surfactants, zwitterionic surfactants, amphoteric surfactants, polymeric surfactants or combinations thereof. Various surfactants for use in the Article are set forth in U.S. Application No. 12/633,228. Each patent described throughout this application is incorporated herein by reference to the extent each provides guidance regarding surfactants suitable for inclusion in the Article.

In one embodiment, the premixture comprises from about 1% to about 75% by weight of the Article of surfactant.

In another embodiment, the Article is a lathering dissolvable solid personal care product (dried) and comprises from about 23% to about 75% by weight of the Article of surfactant, in one embodiment from about 30% to about 70% by weight of the Article of surfactant, in one embodiment from about 40% to about 65% by weight of the Article of surfactant. In such cases, the pre-mixture may comprise from about 8% to about 30% by weight of the pre-mixture of surfactant, in one embodiment from about 13% to about 28% by weight of the pre-mixture of surfactant, in one embodiment from about 18% to about 25% by weight of the pre-mixture of surfactant.

Non-limiting examples of anionic surfactants suitable for use herein include alkyl and alkyl ether sulfates, sulfated monoglycerides, sulfonated olefins, alkyl aryl sulfonates, primary or secondary alkane sulfonates, alkyl sulfosuccinates, acyl taurates, acyl isethionates, alkyl glyceryl ether sulfonate, sulfonated methyl esters, sulfonated fatty acids, alkyl phosphates, acyl glutamates, acyl sarcosinates, alkyl sulfoacetates, acylated peptides, alkyl ether carboxylates, acyl lactylates, anionic fluorosurfactants, sodium lauroyl glutamate, and combinations therof.

Anionic surfactants for use in the Article include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, and combinations thereof.

Amphoteric surfactants suitable for use herein include, but are not limited to derivatives of aliphatic secondary and tertiary amines in which the aliphatic radical can be straight or branched chain and wherein one substituent of the aliphatic substituents contains from about 8 to about 18 carbon atoms and one contains an anionic water solubilizing group, e.g., carboxy, sulfonate, sulfate, phosphate, or phosphonate. Examples include sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of US 2,658,072, N-higher alkyl aspartic acids such as those produced according to the teaching of US 2,438,091, and the products described in US 2,528,378, and mixtures thereof. The family of amphoacetates derived from the reaction of sodium chloroacetate with amidoamines to produce alkanoyl amphoacetates are particularly effective, e.g. lauryolamphoacetate.

Zwitterionic surfactants suitable for use herein include, but are not limited to derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one substituent contains an anionic group, e.g., carboxy, sulfonate, sulfate, phosphate, or phosphonate. Other zwitterionic surfactants suitable for use herein include betaines, including high alkyl betaines such as coco dimethyl carboxymethyl betaine, cocoamidopropyl betaine, cocobetaine, lauryl amidopropyl betaine, oleyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, lauryl bis-(2-hydroxypropyl)alpha-carboxyethyl betaine, and mixtures thereof. The sulfobetaines may include coco dimethyl sulfopropyl betaine, stearyl dimethyl sulfopropyl betaine, lauryl dimethyl sulfoethyl betaine, lauryl bis-(2-hydroxyethyl) sulfopropyl betaine and mixtures thereof. Also suitable amphoteric surfactants include amidobetaines and amidosulfo-betaines, wherein the $RCONH(CH_2)_3$ radical is attached to the nitrogen atom of the betaine are also useful herein.

Cationic surfactants may include a DEQA compound. The DEQA compounds encompass a description of diamido actives as well as actives with mixed amido and ester linkages. DEQA compounds are typically made by reacting alkanolamines such as MDEA (methyldiethanolamine) and TEA (triethanolamine) with fatty acids. Some materials that typically result from such reactions include N,N-di(acyl-oxyethyl)-N,N-dimethylammonium chloride or N,N-di(acyl-oxyethyl)-N,N-methylhydroxyethylammonium methylsulfate wherein the acyl group is derived from animal fats, unsaturated, and polyunsaturated, fatty acids (See US 5,759,990 at column 4, lines 45-66). Additional non-limiting examples of such DEQA compounds are described in US 5,580,481 and US 5,476,597.

Other suitable actives for use as a cationic surfactant include reaction products of fatty acids with dialkylenetriamines in, e.g., a molecular ratio of about 2:1, said reaction products containing compounds of the formula:

$$R^1-C(O)-NH-R^2-NH-R^3-NH-C(O)-R^1$$

wherein $R^1$, $R^2$ are defined as above, and each $R^3$ is a $C_{1-6}$ alkylene group, more specifically an ethylene group. Examples of these actives are reaction products of tallow acid, canola acid, or oleic acids with diethylenetriamine in a molecular ratio of about 2:1, said reaction product mixture containing N,N"-ditallowoyldiethylenetriamine, N,N"-dicanola-oyldiethylenetriamine, or N,N"-dioleoyldiethylenetriamine, respectively, with the formula:

$$R^1-C(O)-NH-CH_2CH_2-NH-CH_2CH_2-NH-C(O)-R^1$$

wherein $R^2$ and $R^3$ are divalent ethylene groups , $R^1$ is defined above and an acceptable examples of this structure when $R^1$ is the oleoyl group of a commercially available oleic acid derived from a vegetable or animal source, include EMERSOL® 223LL or EMERSOL® 7021, available from Henkel Corporation.

Another active for use as a cationic surfactant has the formula:

$$[R^1-C(O)-NR-R^2-N(R)_2-R^3-NR-C(O)-R^1]^+ X^-$$

wherein R, $R^1$, $R^2$, $R^3$ and $X^-$ are defined as above. Examples of this active are the di-fatty amidoamines based softener having the formula:

$$[R^1-C(O)-NH-CH_2CH_2-N(CH_3)(CH_2CH_2OH)-CH_2CH_2-NH-C(O)-R^1]^+CH_3SO_4^-$$

wherein $R^1$-C(O) is an oleoyl group, soft tallow group, or a hardened tallow group available commercially from Degussa under the trade names VARISOFT® 222LT, VARISOFT® 222, and VARISOFT® 110, respectively.

A second type of DEQA ("DEQA (2)") compound suitable as an active for use as a cationic surfactant has the general formula:

$$[R_3N^+CH_2CH(YR^1)(CH_2YR^1)] X^-$$

wherein each Y, R, $R^1$, and $X^-$ have the same meanings as before.

These types of agents and general methods of making them are disclosed in US4,137,180, Naik et al., issued Jan. 30, 1979. An example of a DEQA (2) is the "propyl" ester quaternary ammonium fabric softener active having the formula 1,2-di(acyloxy)-3-trimethylammoniopropane chloride.

Other suitable cationic surfactants can include asymmetric dialkyl quaternized ammonium salt cationic surfactant. The asymmetric dialkyl quaternized ammonium salt cationic surfactant can be included in the composition at a level by weight of in one embodiment from about 0.1% to about 10%, in another embodiment from about 0.2% to about 5%, in yet another embodiment from about 0.4% to about 3% in view of balance between ease-to-rinse feel and wet conditioning benefits. The use of higher level of asymmetric dialkyl quaternized ammonium salt tends to lead to reduced wet conditioning benefits such as reduced slippery feel, while the use of lower level of asymmetric dialkyl quaternized ammonium salt tends to lead to reduced ease-to-rinse feel. Exemplary cationic surfactants and nonionic agents are set forth in U.S. Patent Application U.S. 12/763,286, which is incorporated herein by reference.

In another embodiment, the Article is a substantially non-lathering dissolvable solid personal care product and comprises from about 0% to about 10 % by weight of the Article of an ionic (anionic, zwitterionic, cationic and mixtures thereof) surfactant, in one embodiment from about 0% to about 5% by weight of the Article of an ionic surfactant, and in one embodiment from about 0% to about 2.5% by weight of the Article of an ionic surfactant, and from about 1% to about 50% by weight of the Article of a nonionic or polymeric surfactant, in one embodiment from about 5% to about 45% by weight of the Article of a nonionic or polymeric surfactant, and in one embodiment from about 10% to about 40% by weight of the Article of a nonionic or polymeric surfactant, and combinations thereof.

Suitable nonionic surfactants for use in the present invention include those described in McCutcheon's Detergents and Emulsifiers, North American edition (2010), Allured Publishing Corp., and McCutcheon's Functional Materials, North American edition (2010). Suitable nonionic surfactants for use in the Article of the present invention include, but are not limited to, polyoxyethylenated alkyl phenols, polyoxyethylenated alcohols, polyoxyethylenated polyoxypropylene glycols, glyceryl esters of alkanoic acids, polyglyceryl esters of alkanoic acids, propylene glycol esters of alkanoic acids, sorbitol esters of alkanoic acids, polyoxyethylenated sorbitor esters of alkanoic acids, polyoxyethylene glycol esters of alkanoic acids, polyoxyethylenated alkanoic acids, alkanolamides, N-alkylpyrrolidones, alkyl glycosides, alkyl polyglucosides, alkylamine oxides, and polyoxyethylenated silicones.

In another embodiment, the nonionic surfactant selected from sorbitan esters and alkoxylated derivatives of sorbitan esters including sorbitan monolaurate (SPAN® 20), sorbitan monopalmitate (SPAN® 40), sorbitan monostearate (SPAN® 60), sorbitan tristearate (SPAN® 65), sorbitan monooleate (SPAN® 80), sorbitan trioleate (SPAN® 85), sorbitan isostearate, polyoxyethylene (20) sorbitan monolaurate (Tween® 20), polyoxyethylene (20) sorbitan monopalmitate (Tween® 40), polyoxyethylene (20) sorbitan monostearate (Tween® 60), polyoxyethylene (20) sorbitan monooleate (Tween® 80), polyoxyethylene (4) sorbitan monolaurate (Tween® 21), polyoxyethylene (4) sorbitan monostearate (Tween® 61), polyoxyethylene (5) sorbitan monooleate (Tween® 81), all available from Uniqema, and combinations thereof.

Suitable polymeric surfactants include, but are not limited to, block copolymers of ethylene oxide and fatty alkyl residues, block copolymers of ethylene oxide and propylene oxide, hydrophobically modified polyacrylates, hydrophobically modified celluloses, silicone polyethers, silicone copolyol esters, diquaternary polydimethylsiloxanes, and co-modified amino/polyether silicones.

## B. Polymer

The one or more polymers suitable for the Article herein are selected such that their weighted average molecular weight is from about 40,000 to about 500,000, in one embodiment from about 50,000 to about 400,000, in yet another embodiment from about 60,000 to about 300,000, and in still another embodiment from about 70,000 to about 200,000. The weighted average molecular weight is computed by summing the average molecular weights of each polymer raw material multiplied by their respective relative weight percentages by weight of the total weight of polymers present within the porous solid.

The polymer(s) of the Article can include, but are not limited to, synthetic polymers including polyvinyl alcohols, polyvinylpyrrolidones, polyalkylene oxides, polyacrylates, caprolactams, polymethacrylates, polymethylmethacrylates, polyacrylamides, polymethylacrylamides, polydimethylacrylamides, polyethylene glycol monomethacrylates, copolymers of acrylic acid and methyl acrylate, polyurethanes, polycarboxylic acids, polyvinyl acetates, polyesters, polyamides, polyamines, polyethyleneimines, maleic/(acrylate or methacrylate) copolymers, copolymers of methylvinyl ether and of maleic anhydride, copolymers of vinyl acetate and crotonic acid, copolymers of vinylpyrrolidone and of vinyl acetate, copolymers of vinylpyrrolidone and of caprolactam, vinyl pyrolidone/vinyl acetate copolymers, copolymers of anionic, cationic and amphoteric monomers, and combinations thereof.

The polymer(s) of the Article may also be selected from naturally sourced polymers including those of plant origin examples of which include karaya gum, tragacanth gum, gum Arabic, acemannan, konjac mannan, acacia gum, gum ghatti, whey protein isolate, and soy protein isolate; seed extracts including guar gum, locust bean gum, quince seed, and psyllium seed; seaweed extracts such as Carrageenan, alginates, and agar; fruit extracts (pectins); those of microbial origin including xanthan gum, gellan gum, pullulan, hyaluronic acid, chondroitin sulfate, and dextran; and those of animal origin including casein, gelatin, keratin, keratin hydrolysates, sulfonic keratins, albumin, collagen, glutelin, glucagons, gluten, zein, and shellac.

Modified natural polymers are also useful as polymer(s) in the Article. Suitable modified natural polymers include, but are not limited to, cellulose derivatives such as hydroxypropylmethylcellulose, hydroxymethylcellulose, hydroxyethylcellulose, methylcellulose, hydroxypropylcellulose, ethylcellulose, carboxymethylcellulose, cellulose acetate phthalate, nitrocellulose and other cellulose ethers/esters; and guar derivatives such as hydroxypropyl guar.

Water-soluble polymers of the Article include polyvinyl alcohols, polyvinylpyrrolidones, polyalkylene oxides, starch and starch derivatives, pullulan, gelatin, hydroxypropylmethylcelluloses, methycelluloses, and carboxymethycelluloses.

Water-soluble polymers of the Article also include polyvinyl alcohols, and hydroxypropylmethylcelluloses. Suitable polyvinyl alcohols include those available from Celanese Corporation (Dallas, TX) under the CELVOL trade name including, but not limited to, CELVOL 523, CELVOL 530, CELVOL 540, CELVOL 518, CELVOL 513, CELVOL 508, CELVOL 504, and combinations thereof. Suitable hydroxypropylmethylcelluloses include those available from the Dow Chemical Company (Midland, MI) under the METHOCEL trade name including, but not limited, to METHOCEL E50, METHOCEL E15, METHOCEL E6, METHOCEL E5, METHOCEL E3, METHOCEL F50, METHOCEL K100, METHOCEL K3, METHOCEL A400, and combinations thereof including combinations with above mentioned hydroxypropylmethylcelluloses.

The Article (dried) may comprise from about 10% to about 50% by weight of the Article of a polymer, in one embodiment from about 15% to about 40% by weight of the Article of polymer, in one embodiment from about 20% to about 30% by weight of the Article of a polymer.

The pre-mixture comprises from 0.1% to 25% by weight of the pre-mixture of polymer, in one embodiment from about 5% to about 15% by weight of the pre-mixture of polymer, in one embodiment from about 7% to about 10% by weight of the pre-mixture of polymer.

## C. Plasticizer

The Article may comprise a water soluble plasticizing agent suitable for use in compositions discussed herein. Non-limiting examples of suitable plasticizing agents include polyols, copolyols, polycarboxylic acids, polyesters and dimethicone copolyols.

Examples of useful polyols include, but are not limited to, glycerin, diglycerin, propylene glycol, ethylene glycol, butylene glycol, pentylene glycol, cyclohexane dimethanol, hexane diol, polyethylene glycol (200-600), sugar alcohols such as sorbitol, manitol, lactitol and other mono- and polyhydric low molecular weight alcohols (e.g., $C_2$-$C_8$ alcohols); mono di- and oligo-saccharides such as fructose, glucose, sucrose, maltose, lactose, and high fructose corn syrup solids and ascorbic acid.

Examples of polycarboxylic acids include, but are not limited to citric acid, maleic acid, succinic acid, polyacrylic acid, and polymaleic acid.

Examples of suitable polyesters include, but are not limited to, glycerol triacetate, acetylated-monoglyceride, diethyl phthalate, triethyl citrate, tributyl citrate, acetyl triethyl citrate, acetyl tributyl citrate.

Examples of suitable dimethicone copolyols include, but are not limited to, PEG-12 dimethicone, PEG/PPG-18/18 dimethicone, and PPG-12 dimethicone.

Other suitable platicizers include, but are not limited to, alkyl and allyl phthalates; napthalates; lactates (e.g., sodium, ammonium and potassium salts); sorbeth-30; urea; lactic acid; sodium pyrrolidone carboxylic acid (PCA); sodium hyraluronate or hyaluronic acid; soluble collagen; modified protein; monosodium L-glutamate; alpha & beta hydroxyl acids such as glycolic acid, lactic acid, citric acid, maleic acid and salicylic acid; glyceryl polymethacrylate; polymeric plasticizers such as polyquaterniums; proteins and amino acids such as glutamic acid, aspartic acid, and lysine; hydrogen starch hydrolysates; other low molecular weight esters (e.g., esters of $C_2$-$C_{10}$ alcohols and acids); and any other water soluble plasticizer known to one skilled in the art of the foods and plastics industries; and mixtures thereof.

Plasticizers include glycerin and propylene glycol. EP 0283165 B1 discloses other suitable plasticizers, including glycerol derivatives such as propoxylated glycerol.

The pre-mixture may comprise from about 0.1% to about 25% by weight of the pre-mixture of plasticizer, in one embodiment from 1 to 15 % by weight of the pre-mixture of plasticizer, in one embodiment from about 2% to about 10% by weight of the pre-mixture of plasticizer, in one embodiment from about 2% to about 4% by weight of the pre-mixture of plasticizer.

The Article (dried) may comprise from about 1% to about 25% by weight of the Article of plasticizer, in one embodiment from about 3% to about 20% by weight of the Article of plasticizer, in one embodiment from about 5% to about 15% by weight of the Article of plasticizer.

## D. Optional Ingredients

The Article may further comprise other optional ingredients that are known for use or otherwise useful in compositions, provided that such optional materials are compatible with the selected essential materials described herein, or do not otherwise unduly impair product performance.

Such optional ingredients are most typically those materials approved for use in cosmetics and that are described in reference books such as the CTFA Cosmetic Ingredient Handbook, Second Edition, The Cosmetic, Toiletries, and Fragrance Association, Inc. 1992.

Emulsifiers suitable as an optional ingredient herein include mono- and di-glycerides, fatty alcohols, polyglycerol esters, propylene glycol esters, sorbitan esters and other emulsifiers known or otherwise commonly used to stabilized air interfaces, as for example those used during preparation of aerated foodstuffs such as cakes and other baked goods and confectionary products, or the stabilization of cosmetics such as hair mousses.

Further non-limiting examples of such optional ingredients include preservatives, perfumes or fragrances, coloring agents or dyes, conditioning agents, hair bleaching agents, thickeners, moisturizers, emollients, pharmaceutical actives, vitamins or nutrients, sunscreens, deodorants, sensates, plant extracts, nutrients, astringents, cosmetic particles, absorbent particles, adhesive particles, hair fixatives, fibers, reactive agents, skin lightening agents, skin tanning agents, antidandruff agents, perfumes, exfoliating agents, acids, bases, humectants, enzymes, suspending agents, pH modifiers, hair colorants, hair perming agents, pigment particles, anti-acne agents, antimicrobial agents, sunscreens, tanning agents, exfoliation particles, hair growth or restorer agents, insect repellents, shaving lotion agents, co-solvents or other additional solvents, and similar other materials.

Suitable conditioning agents include high melting point fatty compounds, silicone conditioning agents and cationic conditioning polymers. Suitable materials are discussed in US 2008/0019935, US 2008/0242584 and US 2006/0217288.

Non-limiting examples of product type embodiments for use by the Article include hand cleansing substrates, hair shampoo or other hair treatment substrates, body cleansing substrates, shaving preparation substrates, fabric care substrate (softening), dish cleaning substrates, pet care substrates, personal care substrates containing pharmaceutical or other skin care active, moisturizing substrates, sunscreen substrates, chronic skin benefit agent substrates (e.g., vitamin-containing substrates, alpha-hydroxy acid-containing substrates, etc.), deodorizing substrates, fragrance-containing substrates, and so forth.

**Test Methods**

**A. Distance to Maximum Force Method:**

Measured via a Rupture Method on a Texture Analyzer using a TA-57R cylindrical probe with Texture Exponent 32 Software. The Article should have a thickness of between 4 to 7 mm and cut in a circle with a diameter of at least 7 mm for this method; or carefully cut or stacked to be within this overall thickness and diameter range. The porous solid sample is carefully mounted on top of the cylinder with four screws mounted on top with the top lid affixed in place on top of the sample. There is a hole in the center of the cylinder and its lid which allows the probe to pass through and stretch the sample. The sample is measured with a pre-test speed of 1 mm per second, a test speed of 2 mm per second and a post test speed of 3 mm per second over a total distance of 30 mm. The distance to maximum force is recorded.

**B. Hand Dissolution Method:**

One Article, with dimensions of approximately 43 mm x 43 mm x 4-6 mm, is placed in the palm of the hand while wearing nitrile gloves. 7.5 $cm^3$ of from about 30°C to about 35°C tap water is quickly applied to the product via syringe. Using a circular motion, palms of hands are rubbed together 2 strokes at a time until dissolution occurs (up to 30 strokes). The hand dissolution value is reported as the number of strokes it takes for complete dissolution or as 30 strokes as the maximum.

**C. Lather Profile: Lather Volume:**

The Article provides a lather profile as described hereafter. The lather volume assessment is performed on 15g/10 inch flat Oriental virgin hair switches that have been treated with 0.098g of artificial liquid sebum [10-22% olive oil, 18-20% coconut oil, 18-20% oleic acid, 5-9% lanolin,, 5-9% squalene, 3-6% palmitic acid, 3-6% paraffin oil, 3-6% dodecane, 1-4% stearic acid, 1-4% cholesterol, 1-4% coconut fatty acid, 18-20% choleth-24]. The hair switch is rinsed with 9-11 grain, 100°F water at 1.5 gallons/min for 20 seconds with a shower nozzle. For testing the liquid control products, 0.75 $cm^3$ of liquid product are applied to the center of the switch, the lower portion of hair on the switch is then rubbed over the product on the hair 10 times in a circular motion, followed by 40 strokes back and forth (a total of 80 strokes). Lather speed is recorded as the number of strokes when the first lather is obviously generated during the 80 strokes. Lather from operator's gloves is transferred to a graduated cylinder with a 3.5 cm inside diameter and with total capacities of either 70 ml, 110 ml, or 140 ml depending on the total amount of lather generated (height modification of standard sized graduated cylinders via a glass shop).. Lather from hair is gathered using one downward stroke on the switch with a tight grip and is also placed into the cylinder. Total lather volume is recorded in milliliters. Three runs per test sample are performed and the mean of the three values is calculated. When testing the Article, 0.20 +/- 0.01 grams of product are weighed with the aid of scissors if required and applied to the switch and then 2 $cm^3$ of additional water are added to the product via syringe. The lathering technique is then performed as described for liquid products after a 10 second waiting time.

As used herein, the terms "substantially non-lathering" and "non-lathering" are used to mean a lather volume of from 0 ml to 20 ml.

**D. Cell Wall Thickness**

The Article has a maximum Cell Wall Thickness. The Article has a Cell Wall Thickness of from about from about of from about 15 microns to about 55 microns, in another embodiment from about 20 microns to about 45 microns, and in another embodiment from about 25 microns to about 35 microns.

The Cell Wall Thickness is computed from the scanned images via a micro computed tomography system (μCT80, SN 06071200, Scanco Medical AG) as described herein. The Cell Wall Thickness is determined according to the method defined for the measurement of Trabecular Thickness using Scanco Medical's Bone Trabecular Morphometry evaluation.

The Cell wall thickness and spacing is calculated as the trabecular thickness and trabecular spacing using the ImageJ program with BoneJ plugin. ImageJ is a public domain, Java-based image-processing program developed at the National Institutes of Health and is available for download at http://rsb.info.nih.gov/ij. Information on the development of BoneJ can be found in the following article: Doube M, Klosowski MM, Arganda-Carreras I, Cordelières F, Dougherty RP, Jackson J, Schmid B, Hutchinson JR, Shefelbine SJ. (2010) BoneJ: free and extensible bone image analysis in ImageJ. Bone 47:1076-9. doi: 10.1016/j.bone.2010.08.023.

BoneJ is an open source/free software plugin for ImageJ to facilitate calculations commonly used in trabecular bone analysis. Images obtained from the Scanco μCT50 have a pixel size equal to 0.002mm. These images are subsampled to 0.004mm sized pixels for easier data handling and prepared as a series of binary images (slices) using the program, Aviso Standard v6.3.1. Once the binary images are created, they are exported as a series of 2D TIFF images. The images are then loaded into ImageJ using the "Import Image Sequence" function. They are then analyzed using the BoneJ "Thickness" measurement option. The resulting data has units of pixels and are converted to millimeters by multiplying each data by 0.004.

### E. Specific Surface Area

The Article also has a minimum Specific Surface Area. The Article has a Specific Surface Area of from about 0.03 $m^2/g$ to about 0.25 $m^2/g$, in one embodiment from about 0.035 $m^2/g$ to about 0.22 $m^2/g$, in another embodiment from about 0.04 $m^2/g$ to about 0.19 $m^2/g$, and in still another embodiment from about 0.045 $m^2/g$ to about 0.16 $m^2/g$.

The Specific Surface Area is measured via a gas adsorption technique. Surface Area is a measure of the exposed surface of a solid sample on the molecular scale. The BET (Brunauer, Emmet, and Teller) theory is the most popular model used to determine the surface area and is based upon gas adsorption isotherms. Gas Adsorption uses physical adsorption and capillary condensation to measure a gas adsorption isotherm. The technique is summarized by the following steps; a sample is placed in a sample tube and is heated under vacuum or flowing gas to remove contamination on the surface of the sample. The sample weight is obtained by subtracting the empty sample tube weight from the combined weight of the degassed sample and the sample tube. The sample tube is then placed on the analysis port and the analysis is started. The first step in the analysis process is to evacuate the sample tube, followed by a measurement of the free space volume in the sample tube using helium gas at liquid nitrogen temperatures. The sample is then evacuated a second time to remove the helium gas. The instrument then begins collecting the adsorption isotherm by dosing krypton gas at user specified intervals until the requested pressure measurements are achieved. Samples may then analyzed using an ASAP 2420 with krypton gas adsorption. It is recommended that these measurements be conducted by Micromeretics Analytical Services, Inc. (One Micromeritics Dr, Suite 200, Norcross, GA 30093). More information on this technique is available on the Micromeretics Analytical Services web sites (www.particletesting.com or www.micromeritics.com), or published in a book, "Analytical Methods in Fine particle Technology", by Clyde Orr and Paul Webb.

### F. Thickness

In one embodiment the Article is a flat, flexible substrate in the form of a pad, a strip, or tape and having a thickness of from about 0.5 mm to about 10 mm, in one embodiment from about 1 mm to about 9 mm, in another embodiment from about 2 mm to about 8 mm, and in a further embodiment from about 3 mm to about 7 mm as measured by the below methodology. In another embodiment, the Article is a sheet having a thickness from about 5mm to about 6.5mm. In another embodiment, two or more sheets are combined to form an Article with a thickness of about 5mm to about 10mm.

The thickness of the dissolvable porous solid (i.e., substrate or sample substrate) is obtained using a micrometer or thickness gage, such as the Mitutoyo Corporation Digital Disk Stand Micrometer Model Number IDS-1012E (Mitutoyo Corporation, 965 Corporate Blvd, Aurora, IL, USA 60504). The micrometer has a 1 inch diameter platen weighing about 32 grams, which measures thickness at an application pressure of about 0.09 psi (6.32 gm/cm$^2$).

The thickness of the dissolvable porous solid is measured by raising the platen, placing a section of the sample substrate on the stand beneath the platen, carefully lowering the platen to contact the sample substrate, releasing the platen, and measuring the thickness of the sample substrate in millimeters on the digital readout. The sample substrate should be fully extended to all edges of the platen to make sure thickness is measured at the lowest possible surface pressure, except for the case of more rigid substrates which are not flat. For more rigid substrates which are not completely flat, a flat edge of the substrate is measured using only one portion of the platen impinging on the flat portion of the substrate.

## G. Basis Weight

The Article has a basis weight of from about 200 grams/m$^2$ to about 2,000 grams/m$^2$, in one embodiment from about 400 grams/m$^2$ to about 1,200 grams/m$^2$, in another embodiment from about 600 grams/m$^2$ to about 2,000 grams/m$^2$, and in still another embodiment from about 700 grams/m$^2$ to about 1,500 grams/m$^2$.

The Basis Weight of the dissolvable porous solid component of the personal care composition herein is calculated as the weight of the dissolvable porous solid component per area of the selected dissolvable porous solid (grams/m$^2$). The area is calculated as the projected area onto a flat surface perpendicular to the outer edges of the porous solid. For a flat object, the area is thus computed based on the area enclosed within the outer perimeter of the sample. For a spherical object, the area is thus computed based on the average diameter as 3.14 x (diameter/2)$^2$. For a cylindrical object, the area is thus computed based on the average diameter and average length as diameter x length. For an irregularly shaped three dimensional object, the area is computed based on the side with the largest outer dimensions projected onto a flat surface oriented perpendicularly to this side. This can be accomplished by carefully tracing the outer dimensions of the object onto a piece of graph paper with a pencil and then computing the area by approximate counting of the squares and multiplying by the known area of the squares or by taking a picture of the traced area (shaded-in for contrast) including a scale and using image analysis techniques.

## H. Dry Density

The Article has a dry density of from about 0.08 g/cm$^3$ to about 0.30 g/cm$^3$, in one embodiment from about 0.10 g/cm$^3$ to about 0.25 g/cm$^3$, and in another embodiment from about 0.12 g/cm$^3$ to about 0.20 g/cm$^3$.

The dry density of the dissolvable porous solid is determined by the equation: Calculated Density = Basis Weight of porous solid / (Porous Solid Thickness x 1,000). The Basis Weight and Thickness of the dissolvable porous solid are determined in accordance with the methodologies described herein.

## Scanning Electron Microscope (SEM) Imaging:

Representative sections were cut from the sponge with a clean razor blade and mounted with the cut face up on a standard cryo-SEM stub. Samples were secured onto the stub with carbon tape and silver paint. Samples were imaged using an Hitachi S-4700 FE-SEM fitted with a Gatan Alto 2500 cryo stage. Samples were cooled to -95dC before imaging in the microscope. Samples were lightly coated with Platinum to reduce charging. Representative images were collected at 2kV, 20uA extraction voltage, ultra high resolution mode using the lower secondary electron detector. Long working distances were used to allow the entire sample to be imaged in one frame.

## I. Star Volume and Structure Model Index

The Article has a Star Volume of from about 1 mm$^3$ to about 90 mm$^3$, in one embodiment from about 5 mm$^3$ to about 80 mm$^3$, in another embodiment from about 10 mm$^3$ to about 70 mm$^3$, and in still another embodiment from about 15 mm$^3$ to about 60 mm$^3$. The Article has a non-negative Structure Model Index of from about 0.0 to about 3.0, in one embodiment from about 0.5 to about 2.75, and in another embodiment from about 1.0 to about 2.50.

To measure the cell interconnectivity via the Star Volume and the Structure Model Index, disk-like samples, approximately 4 cm in diameter and 3 to 7 mm high, are scanned using a micro computed tomography system ($\mu$CT80, SN 06071200, Scanco Medical AG). Each sample is imaged while sitting flat on the bottom of a cylindrical tube. Image acquisition parameters are 45 kVp, 177 $\mu$A, 51.2 mm field of view, 800 ms integration time, 1000 projections. The number of slices is adjusted to cover the height of the sample. The reconstructed data set consisted of a stack of images, each 2048x2048 pixels, with an isotropic resolution of 25 $\mu$m. For data analysis, a volume of interest is selected to be fully within the sample, avoiding the surface region. A typical volume of interest is 1028x772x98 voxels.

Structure Model Index (SMI) is measured using Scanco Medical's Bone Trabecular Morphometry evaluation with a threshold of 17. With this index the structural appearance of trabecular bone is quantified (*see* T. Hildebrand, P. Rüegsegger. Quantification of bone microarchitecture with the structure model index. Comp Meth Biomech Biomed Eng 1997;1:15-23). The triangulated surface is dilated in normal direction by an infinitesimal amount, and the new bone surface and volume is calculated. By this, the derivative of the bone surface (dBS/dr) can be determined. The SMI is then represented by the equation:

$$SMI = 6 \cdot \frac{BV \cdot \frac{dBS}{dr}}{BS^2}$$

SMI relates to the convexity of the structure to a model type. Ideal (flat) plates have an SMI of 0 (no surface change with dilation of the plates), whereas ideal cylindrical rods have an SMI of 3 (linear increase in surface with dilation of rods). Round spheres have an SMI of 4. Concave structure gives negative dBS/dr, resulting in negative SMI values. Artificial boundaries at the edge of the volume of interest are not included in the calculation and thus suppressed.

In addition to the Scanco Medical Analysis, Star Volume measurements are made. Star Volume is a measure of the "openness" of the void space in a two phase structure. By choosing a random uniformly distributed set of points in the phase of interest (in this case the phase of interest is the void space or air), lines can be extended in random directions from each of these points. The lines are extended until they touch the foreground phase. The length of each of these lines is then recorded. The random points have a sampling of 10 in each direction (x/y/z) and at each point 10 random angles are chosen. If the line extends to the border of the ROI of interest that line is discarded (only accept lines that actually intersect with the foreground phase). The final equation is based upon the research entitled Star Volume In Bone Research A Histomorphometric Analysis Of Trabecular Bone Structure Using Vertical Sections; Vesterby, A.;Anat Rec.; 1993 Feb;235(2):325-334.:

$$StarVolume = \frac{4}{3}\pi \cdot \frac{\sum dist^3}{N}$$

where "dist" is the individual distances and N is the number of lines examined.

## J. Open Cell Content

The Article has a Percent Open Cell Content of from 80% to 100%, in one embodiment from about 85% to about 97.5%, and in another embodiment from about 90% to about 95%.

The Percent Open Cell Content is measured via gas pycnometry. Gas pycnometry is a common analytical technique that uses a gas displacement method to measure volume accurately. Inert gases, such as helium or nitrogen, are used as the displacement medium. The sample of the Article is sealed in the instrument compartment of known volume, the appropriate inert gas is admitted, and then expanded into another precision internal volume. The pressure before and after expansion is measured and used to compute the sample Article volume. Dividing this volume into the sample Article weight gives the gas displacement density.

## K. Top Surface Bubble Size

The Article has an average top surface bubble size of from about 80 microns to about 250 microns, in another embodiment from about 100 microns to about 220 microns, and in another embodiment from about 120 microns to about 190 microns.

The top surface bubble sizes are measured using the Olympas SZX12 (SN 0100100638) Stereoscope. The resulting images are analyzed using commercially available image analysis software such as the Able Image Analyzer software (MU Labs, SI-1000 Ljubljana, Slovenia, EU). It is understood that the size of bubbles present at the surface of the porous dissolvable substrate are significantly smaller than internal bubble sizes, but the relative differences in surface bubble sizes correlate to the relative differences in internal pore sizes between differing open-celled porous substrates.

## L. Average Pore Size

The Article has an average pore size of from 150 microns to 450 microns, in another embodiment from about 175 microns to about 350 microns, and in another embodiment from about 200 microns to about 300 microns.

The cell wall thickness and the related average pore size is calculated as the trabecular thickness and spacing using the ImageJ program with BoneJ plugin as described in detail above. ImageJ is a public domain, Java-based image-processing program developed at the National Institutes of Health. BoneJ was developed as an open source/free software plugin for ImageJ to facilitate calculations commonly used in trabecular bone analysis.

## Examples

The following examples further describe and demonstrate embodiments within the scope of the present invention. All exemplified amounts are concentrations by weight of the total composition, i.e., wt/wt percentages, unless otherwise specified.

### Example 1: Preparation of Article with Continuous Process

The following surfactant/polymer liquid processing composition is prepared at the indicated weight percentages as described in Table 1 below.

**Table 1**

| Materials | %w/w |
|---|---|
| De-ionized water | 26.4820 |
| Glycerin | 3.2392 |
| Sodium Laureth-3-Sulfate (28% Active) | 4.3878 |
| Mackam HPL-28ULS Sodium Lauroamphoacetate (LAA 26% Active) | 31.8473 |
| Sodium C11AS (35% Active) | 15.9109 |
| Citric Acid (Anhydrous) | 1.5978 |
| UCARE Polymer LR-400 | 0.5000 |
| Celvol 523 | 8.0757 |
| Sodium Laureth-1-Sulfate (SLE1S) (70% Active) | 7.9560 |
| D&C Yellow #10 Dye | 0.0033 |

An Article is prepared from the above liquid processing mixture as described in Table 2 below via a continuous Oakes aerator and dried within an impingement oven according to the following settings and conditions:

**Table 2**

| | Ex 1 |
|---|---|
| Premix Mass Flow Rate w/FAM 10" Die | 39.4 g/min |
| Oakes Air Flow Meter Setting | 56 |
| Oakes RPM | 1941 |
| Wet Density (g/cm$^3$) | 0.25 |
| Impingement Oven Temperature (°C) | 132 |
| Drying Time (min) | 17.9 |
| Average dry substrate weight (g) | 1.1 |
| Average dry substrate density (g/cm$^3$) | 0.11 |
| Average basis weight (g/m$^2$) | 594 |

### Example 2: Comparative Batch Formula/Process

A formula and batch process is used where a surfactant/polymer liquid composition is prepared. The substrate formed by batch processing has the following properties:

**Table 3**

| | Ex2 |
|---|---|
| Aeration Time (sec) | 60 |
| Wet Density (g/cm$^3$) | 0.26 |
| Oven Temperature (°C) | 130 |

(continued)

|  | Ex2 |
| --- | --- |
| Drying Time (min) | 40 |
| Average dry substrate weight (g) | 1.06 |
| Average dry substrate density (g/cm$^3$) | 0.11 |
| Average basis weight (g/m$^2$) | 572 |

**Example 3: Comparison of an Article made from a Continuous Process (Example 1) with an Article made from a Batch Process (Example 2)**

The Articles made from Example 1 and Example 2 are compared. As shown below, Tables 4 and 5 summarize the structural measurements taken on the Articles of Example 1 and Example 2 respectively. SEM and micro-CT images are also taken for Example 1 and Example 2 and are referenced in the attached figures. Trabecular Thickness and Trabecular Spacing are calculated per mm in the z-direction of Example 1 and Example 2 described herein. These calculations are made using the ImageJ program with BoneJ plugin.

**Table 4**

| Example 1 Depth (mm from top) | Micro-CT Cell Wall thickness (mm) | Micro-CT Cell Diameter (mm) | Pycnometry (% Open Cell) | SEM Image | Micro-CT Image |
| --- | --- | --- | --- | --- | --- |
| 1 | 0.0165 | 0.1265 | 96.7 | Fig1 | Fig2 |
| 2 | 0.0183 | 0.1697 | | | |
| 3 | 0.0243 | 0.2619 | | | |
| 4 | 0.0316 | 0.3162 | | | |
| 5 | 0.0403 | 0.3308 | | | |
| 6 | 0.0347 | 0.2926 | | | |
| 6.5 | 0.0247 | 0.1676 | | | |

**Table 5**

| Example 2 Depth (mm from top) | Micro-CT Cell Wall thickness (mm) | Micro-CT Cell Diameter (mm) | Pycnometry (% Open Cell) | SEM Image | Micro-CT Image |
| --- | --- | --- | --- | --- | --- |
| 1 | 0.0406 | 0.3336 | 96.8 | Fig3 | Fig4 |
| 2 | 0.0473 | 0.4620 | | | |
| 3 | 0.0555 | 0.3086 | | | |
| 4 | 0.0860 | 0.4876 | | | |
| 5 | 0.0842 | 0.5648 | | | |
| 6 | 0.0568 | 0.5729 | | | |
| 6.5 | 0.0456 | 0.4960 | | | |

The above data demonstrates the Article of Example 1 as being predominantly open-celled with an average cell size of 0.238mm and an average cell wall thickness of 0.027mm. Correspondingly, the predominantly open-celled Article also exhibits fast dissolution performance (6 to 8 strokes) within the simulated hand dissolution protocol as described herein. The above data also shows the Article of Example 2 to be predominantly open-celled and with an average cell size of 0.461mm and an average cell wall thickness of 0.059mm. Correspondingly, the predominantly open-celled Article also exhibits fast dissolution performance (6 to 8 strokes) within the simulated hand dissolution protocol as described herein. Example 1 demonstrates that the above referenced process results in an open-celled substrate with significant structural differences when compared to the open-celled substrate of Example 2.

### Example 4: Preparation of Article with Continuous Process with Higher Density.

An Article with a higher density is prepared from the same liquid processing composition as described in Table 1 above, and with the same continuous processing. The only difference is that the Article is prepared with a higher density and basis weight by reducing the gas flow rate and/or the rpm as indicated in the table below:

**Table 6**

|  | Ex 1 |
| --- | --- |
| Premix Mass Flow Rate w/FAM 10" Die |  |
| Oakes Air Flow Meter Setting |  |
| Oakes RPM |  |
| Wet Density (g/cm$^3$) |  |
| Impingement Oven Temperature (°C) | 132 |
| Drying Time (min) |  |
| Average dry substrate weight (g) | 1.56 |
| Average dry substrate density (g/cm$^3$) | 0.16 |
| Average basis weight (g/m$^2$) | 850 |

The dissolution data for this sample is given in the table below:

**Table 7**

| Basis Weight (g/m2) | Homogeneity | Dissolution |
| --- | --- | --- |
| 850 | 4.5 | 6-8 |

The above dissolvable Article produced from the continuous process as described herein still exhibits fast dissolution performance (6 to 8 strokes) despite having a higher density and basis weight. This dissolution performance is hypothesized to be due to the improved structural properties from the continuous process of the present invention as demonstrated in Example 3.

### Example 5: Preparation of Layered Article With Continuous Process and Varying Bubble Size

Multi-layered Articles are prepared from the same liquid processing composition as described in Table 1 above and with the same continuous processing. The difference is that they are prepared with reduced thickness which decreases the drying time needed. Each individual layer is prepared with the same reduced thicknesses and basis weights according to the same continuous process as described in Example 1. The only difference in preparation is the reduced drying time and differing bubble size caused by varying the rpms as per Table 8 below.

As seen in the tables below, a surprising positive correlation occurs between bubble size and the number of layers able to achieve passing dissolution. This suggests that the mechanism for the stated positive correlation is the ability for water to more easily penetrate larger pores than smaller pores.

The 2000 rpm processed sheet in Table 8 is cut into 43mm x 43mm squares and weighed. The top surface bubble size is measured using an Olympas SZX12 (SN 0100100638) Stereoscope. The resulting images are analyzed using the Able Image Analyzer software referenced herein. It is understood that the size of bubbles present at the surface of the Article is indicative of the overall internal bubble size as demonstrated in Example 3. The layers are assembled as described in Table 8 and tested for dissolution:

**Table 8**

| 2000 rpm Avg Survace Bubble Size | Layers | Basis Weight (g/m2) | Homogeneity | Dissolution | Micro-CT Image |
|---|---|---|---|---|---|
| 82 μm Dia | 5 | 617 | 1.5 | Undissolved | Fig5 |

The 400rpm processed sheet in Table 9 below is cut into 43mm x 43mm squares and weighed. The top surface bubble size is measured using an Olympas SZX12 (SN 0100100638) Stereoscope. The resulting images are analyzed using the Able Image Analyzer software referenced herein. The layers are assembled as described in Table 9 and tested for dissolution:

**Table 9**

| 400 rpm Avg Survace Bubble Size | Layers | Basis Weight (g/m2) | Homogeneity | Dissolution |
|---|---|---|---|---|
| 92 μm Dia | 1 | 141 | 4.5 | 4 |
| | 2 | 287 | 4.0 | 26 |
| | 3 | 438 | 3.0 | Undissolved |
| | 4 | 579 | 1.8 | Undissolved |

The 100rpm processed sheet in Table 10 below is cut into 43mm x 43mm squares and weighed. The top surface bubble size is measured using an Olympas SZX12 (SN 0100100638) Stereoscope. The resulting images are analyzed using the Able Image Analyzer software referenced herein. The layers are assembled as described in Table 10 and tested for dissolution.

**Table 10**

| 100 rpm Avg Survace Bubble Size | Layers | Basis Weight (g/m2) | Homogeneity | Dissolution |
|---|---|---|---|---|
| 112 μm Dia | 1 | 141 | 4.5 | 4 |
| | 2 | 287 | 4.0 | 10 |
| | 3 | 422 | 3.3 | Undissolved |
| | 4 | 590 | 1.8 | Undissolved |

The 50rpm processed sheet in Table 11 is cut into 43mm x 43mm squares and weighed. The top surface bubble size is measured using an Olympas SZX12 (SN 0100100638) Stereoscope. The resulting images are analyzed using the Able Image Analyzer software referenced herein. The layers are assembled as described in Table 11and tested for dissolution:

**Table 11**

| 50 rpm Avg Survace Bubble Size | Layers | Basis Weight (g/m2) | Homogeneity | Dissolution |
|---|---|---|---|---|
| 126 μm Dia | 1 | 135 | 4.5 | 4 |
| | 2 | 276 | 4.5 | 6 |
| | 3 | 422 | 3.5 | 24 |
| | 4 | 557 | 3.0 | Undissolved |

The 20rpm processed sheet in Table 12 is cut into 43mm x 43mm squares and weighed. The top surface bubble size is measured using the Olympas SZX12 (SN 0100100638) Stereoscope. The resulting images are analyzed using the Able Image Analyzer software referenced herein. The layers are assembled as described in Table 12 and tested for dissolution.

**Table 12**

| 20 rpmAvg Survace Bubble Size | Layers | Basis Weight (g/m2) | Homogeneity | Dissolution |
|---|---|---|---|---|
| 150 μm Dia | 1 | 178 | 4.8 | 4 |
| | 2 | 346 | 4.5 | 6 |
| | 3 | 530 | 3.5 | 9 |
| | 4 | 719 | 3.2 | 23 |

### Example 6: Positive Correlation between Pore Size and Dissolution Rate

The aforementioned positive correlation between pores size and dissolution as shown in Example 5 is summarized in Table 13 below:

**Table 13**

| Example | Process Setting (RPM) | Average Surface Bubble Size (mm) | 1 layer Dissolution (strokes) | 2 layers Dissolution (strokes) | 3 layers Dissolution (strokes) | 4 layers Dissolution (strokes) |
|---|---|---|---|---|---|---|
| 5.2 | 400 | 0.092 | 4 | 26 | 30+ | 30+ |
| 5.3 | 100 | 0.112 | 4 | 10 | 30+ | 30+ |
| 5.4 | 50 | 0.126 | 4 | 6 | 24 | 30+ |
| 5.5 | 20 | 0.150 | 4 | 6 | 9 | 23 |

Example 6 demonstrates the importance of having larger pore sizes (quantified via surface bubble size measurements) to achieve improved dissolution performance from a multi-layered Article.

### Example 7: Composition of the Article for a Continuous Process

| Component | Ex. 11 | Ex. 12 | Ex. 13 | Ex. 14 |
|---|---|---|---|---|
| Weight Percent Solids | 30.10% | 33.30% | 34.30% | 35.30% |
| Anionic:Amphoteric (Zwitterionic) Ratio | 60:40:00 | 60:40:00 | 60:40:00 | 60:40:00 |
| Glycerin | 3 | 3.2 | 3.2 | 3.2 |
| Polyvinyl alcohol[1] | 7.4 | 8.1 | 8.1 | 8.1 |
| Sodium Lauroamphoacetate (26% activity)[2] | 29.2 | 31.8 | 31.8 | 31.8 |
| Ammonium Laureth-3 sulfate (25% activity) | 4.5 | 4.9 | 4.9 | 4.9 |
| Ammonium Undecyl sulfate (24% activity) | 18.2 | 19.9 | 19.9 | 19.9 |
| Ammonium Laureth-1 sulfate (70% activity) | 7.3 | 8 | 8 | 8 |
| Cationic cellulose[3] | 0.5 | 0.5 | 0.5 | 0.5 |
| Citric Acid | 0.95 | 1.6 | 1.6 | 1.6 |
| Distilled water | 28.95 | 22 | 18 | 14 |
| Silicone Microemulsion A (25% Activity) | | | 4 | |
| Silicone Microemulsion B (25% Activity) | | | | 8 |
| Total | 100 | 100 | 100 | 100 |
| pH | 6.1 | 5.8 | 5.8 | 5.8 |

(continued)

| Component | Ex. 11 | Ex. 12 | Ex. 13 | Ex. 14 |
|---|---|---|---|---|
| Viscosity (cp) | 19,600 | 35,400 | | |
| Dow Corning 2-1865; Internal Phase Viscosity = 34,000 cps; 30 nm particle size dimethiconol using TEA dodecyl benzene sulfonate and laureth 23 as primary surfactants. | | | | |
| Dow Corning 2-1870; Internal Phase Viscosity = 70,000 cps; 30 nm particle size dimethiconol using TEA dodecyl benzene sulfonate and laureth 23 as primary surfactants. | | | | |

Note that any actives and/or compositions disclosed herein can be used in and/or with the Articles, disclosed in the following U.S Patent Applications, including any publications claiming priority thereto: US 61/229981; US 61/229986; US 61/229990; US 61/229996; US 61/230000; and US 61/230004.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A continuous process for preparing a flexible porous dissolvable solid structure article, comprising the steps of:

   a. preparing a pre-mixture comprising from 1% to 75% by weight based on the total weight of the pre-mixture of surfactant, from 0.1% to 25% by weight based on the total weight of the pre-mixture of polymer, from 0.1% to 75% by weight based on the total weight of the pre-mixture of water, and optionally from 0.1% to 25% by weight based on the total weight of the pre-mixture of plasticizer, wherein said pre-mixture comprises:

   i. a viscosity at 70°C and a shear rate of 1 sec-1 of from 1000 cps to 20,000 cps; and
   ii. wherein said pre-mixture is heated to a temperature in the range of from 60°C to 90°C;

   b. aerating the pre-mixture by introducing a gas into the pre-mixture to form a wet aerated pre-mixture, wherein the pre-mixture is aerated using a roter stater mixer, wherein said wet aerated pre-mixture comprises:

   i. a density of from 0.15 to 0.65 g/ml; and
   ii. a bubble size of from 20 to 80 microns;

   c. extruding the wet aerated pre-mixture to form one or more sheets on a belt; and
   d. drying the sheets to form the article wherein the article is an open celled foam with a % open cell of from 80% to 100%, wherein the drying environment is an impingement oven, such that the drying environment is between 100°C and 150°C.

2. The process of claim 1, wherein article has an average top surface bubble size from 20 microns to 250 microns.

3. The process of any of the preceding claims, wherein the belt is composed of a solid metallic material, a flexible plastic material, and combinations thereof.

4. The process of claim 3, wherein the flexible plastic material is an infrared transparent material.

5. The process of any of the preceding claims, wherein the article has a basis weight of from 200 grams/m$^2$ to 2,000 grams/m$^2$.

6. The process of any of the preceding claims, wherein two or more sheets are combined to form an article having a thickness from 5.0 mm to 10 mm.

7. The process of any of the preceding claims, further comprising a heating step of the pre-mixture before and during the aeration process wherein the pre-mixture is between 40°C and 99°C.

8. The process of any of the preceding claims, wherein the drying step results in sheets with a moisture content of 5% to 10%.

9. The process of any of the preceding claims, wherein the article comprises a surface area of from 0.03 $m^2$/g to 0.25 $m^2$/g.

10. The process of any of the preceding claims, wherein the pre-mixture is a microemulsion.

11. The process of any of the preceding claims, wherein the article comprises a distance to maximum force value of from 6mm to 30mm.

12. The process of any of the preceding claims, further comprising a step of modifying the article selected from the group consisting of: forming the article into a three dimensional structure, printing onto the surface of the article, texturizing the surface of the article and combinations thereof.

13. A flexible porous dissolvable solid structure article produced by a continuous process as claimed in any of the preceding claims, wherein the article has an average pore size of from 150 microns to 450 microns, wherein the Article has a Percent Open Cell Content of from about 80% to 100%.

**Patentansprüche**

1. Kontinuierliches Verfahren zur Herstellung eines Gegenstands mit elastischer, poröser, auflösbarer, fester Struktur, umfassend die folgenden Schritte:

   a. Herstellen einer Vormischung, umfassend von 1 Gew.-% bis 75 Gew.-% (basierend auf dem Gesamtgewicht der Vormischung) Tensid, von 0,1 Gew.-% bis 25 Gew.-% (basierend auf dem Gesamtgewicht der Vormischung) Polymer, von 0,1 Gew.-% bis 75 Gew.-% (basierend auf dem Gesamtgewicht der Vormischung) Wasser und wahlweise von 0,1 Gew.-% bis 25 Gew.-% (basierend auf dem Gesamtgewicht der Vormischung) Weichmacher, wobei die Vormischung umfasst:

      i. eine Viskosität bei 70 °C und eine Scherrate von 1 s-1 von 1000 cP bis 20.000 cP; und
      ii. wobei die Vormischung auf eine Temperatur im Bereich von 60 °C bis 90 °C erwärmt wird;

   b. Belüften der Vormischung durch Einleiten eines Gases in die Vormischung unter Bildung einer nassen, belüfteten Vormischung, wobei die Vormischung mittels eines Rotor-Stator-Mischers belüftet wird, wobei die nasse, belüftete Vormischung umfasst:

      i. eine Dichte von 0,15 bis 0,65 g/ml; und
      ii. eine Blasengröße von 20 bis 80 Mikrometern;

   c. Extrudieren der nassen, belüfteten Vormischung unter Bildung einer oder mehrerer Lagen auf einem Band; und
   d. Trocknen der Lagen, um den Gegenstand zu bilden, wobei der Gegenstand ein offenzelliger Schaum mit einer Offenzelligkeit in % von 80 % bis 100 % ist, wobei die Trocknungsumgebung ein Aufprallofen ist, derart, dass die Trocknungsumgebung zwischen 100 °C und 150 °C liegt.

2. Verfahren nach Anspruch 1, wobei der Gegenstand eine durchschnittliche Oberseiten-Blasengröße von 20 Mikrometern bis 250 Mikrometern aufweist.

3. Verfahren nach einem der vorstehenden Ansprüche, wobei das Band aus einem festen metallischen Material, einem elastischen Kunststoffmaterial und Kombinationen davon besteht.

4. Verfahren nach Anspruch 3, wobei das elastische Kunststoffmaterial ein infrarotdurchlässiges Material ist.

5. Verfahren nach einem der vorstehenden Ansprüche, wobei der Gegenstand ein Basisgewicht von 200 Gramm/$m^2$

bis 2.000 Gramm/m$^2$ aufweist.

**6.** Verfahren nach einem der vorstehenden Ansprüche, wobei zwei oder mehr Lagen kombiniert werden, um einen Gegenstand mit einer Dicke von 5,0 mm bis 10 mm zu bilden.

**7.** Verfahren nach einem der vorstehenden Ansprüche, ferner umfassend einen Schritt des Erwärmens der Vormischung vor und während des Belüftungsverfahrens, wobei die Vormischung zwischen 40 °C und 99 °C liegt.

**8.** Verfahren nach einem der vorstehenden Ansprüche, wobei der Trocknungsschritt zu Lagen mit einem Feuchtigkeitsgehalt von 5 % bis 10 % führt.

**9.** Verfahren nach einem der vorstehenden Ansprüche, wobei der Gegenstand einen Oberflächenbereich von 0,03 m$^2$/g bis 0,25 m$^2$/g aufweist.

**10.** Verfahren nach einem der vorstehenden Ansprüche, wobei die Vormischung eine Mikroemulsion ist.

**11.** Verfahren nach einem der vorstehenden Ansprüche, wobei der Gegenstand eine Distanz zum Maximalkraftwert von 6 mm bis 30 mm umfasst.

**12.** Verfahren nach einem der vorstehenden Ansprüche, ferner umfassend einen Schritt des Modifizierens des Gegenstands, ausgewählt aus der Gruppe bestehend aus: Formen des Gegenstands zu einer dreidimensionalen Struktur, Bedrucken der Oberfläche des Gegenstands, Strukturieren der Oberfläche des Gegenstands und Kombinationen davon.

**13.** Gegenstand mit elastischer, poröser, auflösbarer, fester Struktur, hergestellt durch ein kontinuierliches Verfahren nach einem der vorstehenden Ansprüche, wobei der Gegenstand eine durchschnittliche Porengröße von 150 Mikrometern bis 450 Mikrometern aufweist, wobei der Gegenstand einen Offenzellgehalt in Prozent von etwa 80 % bis 100 % aufweist.

**Revendications**

**1.** Procédé continu pour la préparation d'un article à structure solide soluble poreuse souple, comprenant les étapes consistant à :

a. préparer un prémélange comprenant de 1 % à 75 % en poids sur la base du poids total du prémélange d'agent tensioactif, de 0,1 % à 25 % en poids sur la base du poids total du prémélange de polymère, de 0,1 % à 75 % en poids sur la base du poids total du prémélange d'eau et, facultativement de 0,1 % à 25 % en poids sur la base du poids total du prémélange de plastifiant, dans lequel ledit prémélange comprend :

i. une viscosité à 70 °C et à un taux de cisaillement de 1 s-1 allant de 1000 cP à 20 000 cP ; et
ii. dans lequel ledit prémélange est chauffé à une température dans la plage allant de 60 °C à 90 °C ;

b. aérer le prémélange en introduisant un gaz dans le prémélange pour former un prémélange aéré humide, dans lequel le prémélange est aéré en utilisant un mélangeur à rotor stator, dans lequel ledit prémélange aéré humide comprend :

i. une masse volumique allant de 0,15 à 0,65 g/ml ; et
ii. une taille de bulle allant de 20 à 80 micromètres ;

c. extruder le prémélange aéré humide pour former une ou plusieurs feuilles sur une courroie ; et
d. sécher les feuilles pour former l'article dans lequel l'article est une mousse à alvéoles ouvertes avec un % d'alvéoles ouvertes allant de 80 % à 100 %, dans lequel l'environnement de séchage est un four à chaleur puisée, de telle sorte que l'environnement de séchage est entre 100 °C et 150 °C.

**2.** Procédé selon la revendication 1, dans lequel l'article a une taille moyenne de bulle de surface supérieure allant de 20 micromètres à 250 micromètres.

**3.** Procédé selon l'une quelconque des revendications précédentes, dans lequel la courroie est composée d'un matériau métallique solide, un matériau plastique souple et leurs combinaisons.

**4.** Procédé selon la revendication 3, dans lequel le matériau plastique souple est un matériau transparent aux infrarouges.

**5.** Procédé selon l'une quelconque des revendications précédentes, dans lequel l'article a une masse surfacique allant de 200 grammes/m$^2$ à 2000 grammes/m$^2$.

**6.** Procédé selon l'une quelconque des revendications précédentes, dans lequel deux feuilles ou plus sont combinées pour former un article ayant une épaisseur allant de 5,0 mm à 10 mm.

**7.** Procédé selon l'une quelconque des revendications précédentes, comprenant en outre une étape de chauffage du prémélange avant et pendant le procédé d'aération dans lequel le prémélange se trouve entre 40 °C et 99 °C.

**8.** Procédé selon l'une quelconque des revendications précédentes, dans lequel l'étape de séchage donne des feuilles avec une teneur en humidité de 5 % à 10 %.

**9.** Procédé selon l'une quelconque des revendications précédentes, dans lequel l'article comprend une superficie allant de 0,03 m$^2$/g à 0,25 m$^2$/g.

**10.** Procédé selon l'une quelconque des revendications précédentes, dans lequel le prémélange est une microémulsion.

**11.** Procédé selon l'une quelconque des revendications précédentes, dans lequel l'article comprend une valeur de distance à la force maximale allant de 6 mm à 30 mm.

**12.** Procédé selon l'une quelconque des revendications précédentes, comprenant en outre une étape consistant à modifier l'article, choisi dans le groupe constitué de : formage de l'article en une structure tridimensionnelle, impression sur la surface de l'article, texturation de la surface de l'article et leurs combinaisons.

**13.** Article à structure solide soluble poreuse souple produit par un procédé continu selon l'une quelconque des revendications précédentes, dans lequel l'article a une taille moyenne de pore allant de 150 micromètres à 450 micromètres, dans lequel l'article a une teneur en pour cent d'alvéoles ouvertes allant d'environ 80 % à 100 %.

Fig. 1

Fig. 4

Fig. 3

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20110027328 A
- US 633228
- US 2658072 A
- US 2438091 A
- US 2528378 A
- US 5759990 A
- US 5580481 A
- US 5476597 A
- US 4137180 A, Naik
- US 763286
- EP 0283165 B1
- US 20080019935 A
- US 20080242584 A
- US 20060217288 A
- US 61229981 B
- US 61229986 B
- US 61229990 B
- US 61229996 B
- US 61230000 B
- US 61230004 B

### Non-patent literature cited in the description

- North American edition. McCutcheon's Detergents and Emulsifiers. Allured Publishing Corp, 2010
- North American edition. McCutcheon's Functional Materials. 2010
- CTFA Cosmetic Ingredient Handbook. The Cosmetic, Toiletries, and Fragrance Association, Inc, 1992
- **DOUBE M ; KLOSOWSKI MM ; ARGANDA-CARRERAS I ; CORDELIÈRES F ; DOUGHERTY RP ; JACKSON J ; SCHMID B ; HUTCHINSON JR ; SHEFELBINE SJ.** BoneJ: free and extensible bone image analysis in ImageJ. *Bone,* 2010, vol. 47, 1076-9
- **CLYDE ORR ; PAUL WEBB.** Analytical Methods in Fine particle Technology
- **T. HILDEBRAND ; P. RÜEGSEGGER.** Quantification of bone microarchitecture with the structure model index. *Comp Meth Biomech Biomed Eng,* 1997, vol. 1, 15-23
- **VESTERBY, A. ; ANAT REC.** *Star Volume In Bone Research A Histomorphometric Analysis Of Trabecular Bone Structure Using Vertical Sections,* February 1993, vol. 235 (2), 325-334